# EUROPEAN PATENT SPECIFICATION

(11) **EP 4 011 299 B1**
(45) Date of publication and mention of the grant of the patent: **08.04.2026**
(21) Application number: 20306553.7
(22) Date of filing: 11.12.2020
(51) Int. Cl.: A61B 8/06, A61B 8/08, A61B 8/12, A61B 8/00

(54) **ULTRASOUND DEVICE TRACKING SYSTEM**
VERFOLGUNGSSYSTEM FÜR ULTRASCHALLVORRICHTUNG
SYSTÈME DE SUIVI DE DISPOSITIF À ULTRASONS

(43) Date of publication of application: 15.06.2022
(73) Proprietor: Sorbonne Université, 75006 Paris (FR); Centre National de la Recherche Scientifique, 75016 Paris (FR); INSERM (Institut National de la Santé et de la Recherche Médicale), 75013 Paris (FR)
(72) Inventor: GOUDERT, Antoine, 75015 Paris (FR); COUTURE, Olivier, 92130 Issy-les-Moulineaux (FR); HINGOT, Vincent, 75006 Paris (FR)
(74) Representative: Icosa

(56) References cited:
- EP-A1- 2 399 522
- WO-A1-2016/145439
- WO-A1-2019/238526
- US-A1- 2019 320 984
- VAN SLOUN RUUD J G ET AL: "Super-Resolution Ultrasound Localization Microscopy Through Deep Learning", IEEE TRANSACTIONS ON MEDICAL IMAGING, IEEE SERVICE CENTER, PISCATAWAY, NJ, US, vol. 40, no. 3, 16 November 2020 (2020-11-16), pages 829 - 839, XP011840877, ISSN: 0278-0062, [retrieved on 20210302], DOI: 10.1109/TMI.2020.3037790

## Description

### FIELD OF INVENTION

The present invention relates to ultrasound imaging, in particular ULM imaging and ultrasound communication used in device localization inside a target body structure of a patient.

### BACKGROUND OF INVENTION

Inspired the single molecule localization microscopy in optics which revolutionized cell imaging, Ultrasound Localisation Microscopy (ULM) has recently begun to revolutionize biomedical ultrasound imaging.

It is well known from the state of the art that the resolution in B-mode imaging and Doppler imaging is limited both axially and laterally by the wavelength. The ultimate limit is set by the Rayleigh criterion. ULM overcomes this diffraction barrier and enables to push the resolution - attenuation trade-off further.

The core idea of ULM is knowingly to introduce sparse punctual sources in the medium being imaged, to highlight specific parts. These sources are air microbubbles, more precisely millions of microbubbles, also called contrast agents. Thanks to these microbubbles, the vascular system is resolved under the diffraction barrier. A super-resolved image is constructed by localizing each bubble centre separately and accumulating their positions to recover the vessel's network, several times smaller than the wavelength. The use of microbubbles (with a diameter ranging from 1 to 3 µm), thanks to their high compressibility, allows the imaging system to outperform accuracy limitations due to the classical wave diffraction theory which is around half of the wavelength and to bypass the usual compromise to be found between wave penetration (favoured in the low wav frequency range) and image resolution (favoured in the high wav frequency range). This enables to visualize details which remain invisible on images built by conventional echography, Doppler echography in particular.

In particular regarding brain vascularization, this technology enables the creation of highly precise images enabling a precise mapping of a patient's brain vascular system. In case of a stroke, in the particular case of a thrombectomy operation, this mapping could highly improve the ability of a surgeon to navigate a catheter tip inside the brain's blood vessel. It is well known by any person skilled in the art that just after a stroke, a recent occlusion can be reopened without surgery by thrombolysis, thrombo-aspiration or mechanical thrombectomy, for example. All those techniques necessitate, in at least one of their implementation options, the surgeon to navigate a catheter tip through the brains vascular system in order to reach the occlusion point. Nowadays, the surgeon visualizes the catheter by means of fluoroscopy, a 2D x-ray-based technique that does not allow the 3D tracking of the catheter. This technique leads to a 2-dimensionnal imaging obtained by means of X rays and can be associated to 3-dimensional MRI data. Generally, the surgeon is shown one or two plane fluoroscopy images, which are x-ray projection images, meanwhile (s)he has access to an MRI scan on another screen. The placement of the catheter thus implies to inject X-Ray contrast agents which may comprise some health damaging elements. This leads to well-known localisation and navigation difficulties, as the surgeon lacks a 3D mapping of the brain vascularization, allowing a 3D navigation plan. This is even more important in the brain (with regards to other parts of the body) as the brain includes lots of very tortuous blood vessels which are not well cut out on 2D imaging.

WO 2019/238526 notably discloses a device from the prior art that uses classic ultrasound imagery to generate a tracking of one or several elements located inside a patient's body.

The aim of the current invention is thus to provide an accurate visual localisation of a device inside a target body structure inside a 3D mapping of the target body structure, in order to facilitate the navigation of a device inside this target body part.

### SUMMARY

This invention thus relates to a device tracking system configured to monitor a target body structure of a patient and localize a device inside said target body structure, the tracking system comprising:
- a control unit with a memory, the memory being configured to store at least one ULM, that is Ultrasound Localization Microscopy, ultrasound image of the target body structure, the control unit being configured to launch an acquisition of the least one ULM ultrasound image,
- a device comprising:
   ∘ at least one steering element configured to be handled outside the target body structure,
   ∘ at least one steerable element configured to be introduced inside the target body structure, the steerable element and the steering element being configured to be physically connected by at least one connection element,
- at least one probe configured to be brought in contact with a securing body part of the patient, the securing body part surrounding at least partially the target body structure, the at least one probe being configured to be in real time communication with the control unit,
- at least one tracker configured to be secured to the steerable element of the device,

wherein the at least one probe and the at least one tracker communicate by means of ultrasounds and information collected as a result of this ultrasound communication is sent to the control unit, the control unit being thus able to localize, in real time, the steerable element inside the target body structure,
wherein the control unit is further designed to display, on a screen, the at least one stored ULM ultrasound image and display, in real time, the localisation of the at steerable element on said at least one ultrasound image.

This way, the solution enables to reach the here-above mentioned objective. Especially, the use of the tracking system enables an improved precision and thus a quicker and safer interventional response from the surgeon, in particular after a stroke, in order to minimize the impact of said stroke, particularly regarding occlusions.

The stroke scanner according to the invention may comprises one or several of the following features, taken separately from each other or combined with each other:
- the memory of the control unit is configured to store a succession of ULM ultrasound image of the target body structure, each new ULM ultrasound image replacing the prior one,
- the acquisition of an ULM ultrasound image is done in real-time, a new acquisition of an ULM ultrasound image being launched as soon the prior acquisition of an ULM ultrasound image is terminated, each new ULM ultrasound image replacing the prior one as soon its acquisition is terminated,
- a new acquisition of an ULM ultrasound image is launched, by the control unit as soon the prior acquisition of an ULM ultrasound image is terminated,
- the control unit is configured to combine the real-time information collected as a result of the ultrasound communication and the information of the stored ULM ultrasound image,
- the control unit is an emission/reception system configured to transform electric impulses into acoustic impulses and vice versa in order to enable acoustic characterization of a given environment, in particular the target body structure,
- the at least one probe may comprise at least one ultrasound transducer and the at least one tracker may comprise at least one ultrasound sensor,
- the at least one probe is configured to be removably secured to the securing body part of the patient, the securing body part surrounding at least partially the target body structure,
- the at least one tracker may comprise an object strongly reflecting ultrasounds waves,
- the at least one tracker may comprise at least one ultrasound transducer and the at least one probe may comprise at least one ultrasound sensor,
- the at least one steerable element may measure less than 1cm in diameter, more particularly between 0,1 and 3mm in diameter,
- the device may be a catheter, the at least one steerable element may be a catheter tip, and the at least one steering element may be a catheter handle,
- the target body structure may be the vascular system of the brain,
- the control unit is able to localize the steerable element with a better precision than half the size of the wavelength of the ultrasound used to perform the localization.

The system may further be for use in thrombectomy.

### BRIEF DESCRIPTION OF THE DRAWINGS

**Figure 1** is a ULM image of a target body structure,
**Figure 2** is a schematic view of the one embodiment of the system according to the present invention,
**Figure 3** is a schematic view of the device inserted in the target body structure according to the present invention,
**Figure 4** is an example of a visualization obtained by means of the device according to the present invention.

### DETAILED DESCRIPTION

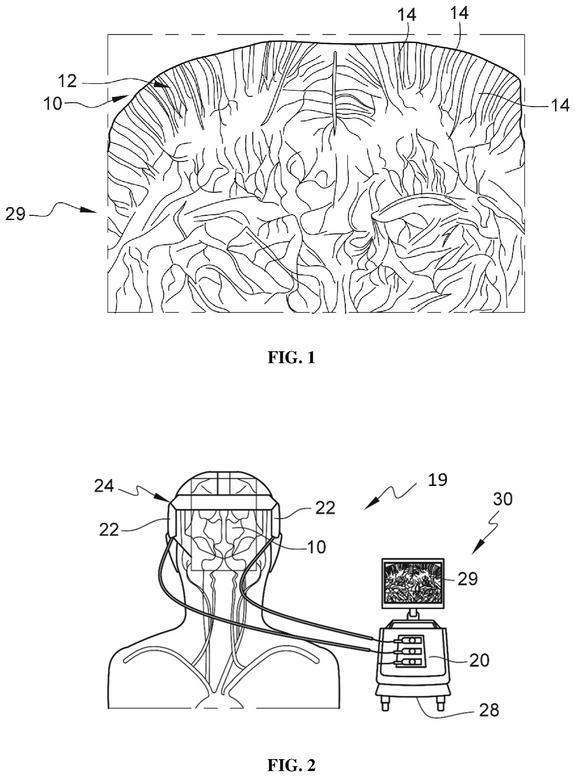

As can be seen on figure 1, a typical target body structure 10 like a brain vascular system 12 counts an amazingly high number of blood vessels 14. It is well known from any person skilled in the art that the normal function of the brain's control centres (for example, but this is true for any organ in the body) depends upon adequate oxygen and nutrients supply through a dense network of blood vessels 14. Blood is usually supplied to the brain, face, and scalp via two major sets of vessels 14: the right and left common carotid arteries and the right and left vertebral arteries. The common carotid arteries are well known to display two divisions:
- the external carotid arteries supply the face and scalp with blood,
- the internal carotid arteries supply blood to most of the anterior portion of the cerebrum,
- the vertebrobasilar arteries supply the posterior two-fifths of the cerebrum, part of the cerebellum, and the brain stem.

Any decrease in the flow of blood through one of the internal carotid arteries brings about some impairment in the function of the frontal lobes. This impairment may result in numbness, weakness, or paralysis on the side of the body opposite to the obstruction of the artery. Even worse, occlusion of one of the vertebral arteries can cause many serious consequences, ranging from blindness to paralysis, or death, in millions of cases per year.

It is therefore essential to intervene as soon as
possible to provide blood flow to these ischemic areas. One treatment is thrombolysis (tPA), which can only be injected within the first few hours after the onset of symptoms.

Another option is thrombectomy, where a catheter is introduced in the vascular system and inserted up to the thrombus in the artery to mechanically remove it.

Occlusions are thus well known to be treated by means of a device, for example a catheter, introduced in the damaged blood vessel 14. Regarding the present invention, such a device 15 comprises at least one steerable element 16 and at least one steering element 17. The steering element 17 is handled from outside the target body structure 10 by a surgeon or a robotic device, for example. This steering element 17 enables the mechanical steering and positioning of the steerable element 16 which is aimed at being introduced inside the target body structure 10. The steering element 17 thus enables the direct manually or robotic steering of the steerable element 16. The steerable element 16 and the steering element 17 are thus physically connected by at least one physical connection element 18. The steerable element 16, each steering element 17 and the connection element 18 can all be produced in one piece or can be removable secured to each other. In the case where said device 15 is a catheter, the steerable element 16 is the catheter tip, the steering element 17 is a catheter handle and the connection elements 18 form the catheter body. In case the device 15 is a catheter, the steerable element 16 is introduced through the intra-femoral artery inside the patient's brain vascular system 12 and manually navigated, by means of the steering element 17, through said vascular system 12 until the occlusion point of the damaged vessel 14 is reached. In order to help navigating the steerable element 16 (in the present example, the catheter tip) through the vascular system 12, the surgeon needs visual help. This visual help is usually displayed on a screen and necessitates to monitor the inserted steerable element 16.

According to the present invention, and as can be seen on figure 2, this monitoring is done by means of a tracking system 19 aimed at monitoring the target body structure 10 of the patient. This system 19 according to the current invention comprises:
- a control unit 20 with a memory,
- at least one probe 22, more particularly an ultrasound probe, aimed at being removably secured to a securing body part 24 of the patient, the securing body part 24 surrounding at least partially the target body structure 10,
- at least one tracker 26 aimed at being secured to the steerable element 15 and thus aimed at being introduced inside the target body structure 10.

The control unit 20 is an emission/reception system which transforms electric impulses into acoustic impulses (and vice versa) in order to enable the acoustic characterization of a given environment, in this case, the target body structure 10.

In the embodiment illustrated on figure 2, the system 19 includes two probes 22, each secured to a temple of the patient. The securing body part 24 in this embodiment is thus the forehead of the patient. In some other embodiments, not shown, there could be more probes, secured on different securing body parts 24. In some other embodiments, a probe might be handheld.

The probe 22 are brought in contact with the securing body part 24. Even in case a gel is spread between the securing body part 24 and the probe 22, it is considered that the probe is in contact with the securing body part 24. The probe could be manually handled. In another embodiment, the probes 22 are secured to the securing body part 24 for example by means of a helmet or an elastic holder, as can be seen on figure 2. The probes 22 have to be easily, quick and removably secured to the securing body part 24 of the patient. In some embodiments, each probe 22 comprises at least one ultrasound transducer, for example a piezo-electric transducer. In some other embodiments, each probe 22 comprises at least one ultrasound sensor, preferably three sensors in order to be able to localize the tracker 26 by means of triangulation. Each probe 22 is in real time communication with the control unit 20.

In the current application, the term "transducer" is used synonymously as "emitter" and the term "sensor" is used synonymously as "receptor".

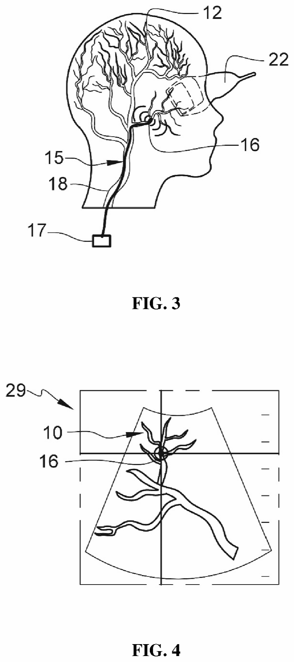

According to the embodiment illustrated on figure 3, the at least one tracker is secured to the steerable element 16 of the device 15, which steerable element 16 can be manually introduced inside the target body structure 10 by means of the steering element 17 and the connection elements 18. This kind of steerable elements 16 usually measures less than 1cm in diameter, more particularly between 0,1 and 3mm in diameter. In some embodiments, each at least one second tracker comprises at least one ultrasound transducer, for example a piezo-electric transducer. In some other embodiments, the at least one second tracker comprises at least one ultrasound sensor, for example a strongly reflective object.

In every embodiment, the probe 22 and the tracker communicate by means of ultrasounds, regardless of which ones are the transducer(s) or the sensor(s). In the case of the tracker used as sensor, there is no need to generate high-voltages inside the device 15. The device 15 is thus a passive device. In the case of the tracker used as a transducer (emitter), it allows better signal-to-noise ratio in the positioning. The information collected by each probe 22 from the at least one tracker is sent, in real time, to the control unit 20. The control unit 20 is thus able to localize, in real time, the at least one tracker inside the target body structure 10. Depending on the ultrasound technology used, the control unit 20 is able to localize the steerable element with a better precision than half the size of the wavelength of the ultrasound used to perform the localization.

The control unit 20 further comprises a memory 28 aimed at storing at least one ULM ultrasound image 29 of the target body structure 10, like for example the image illustrated on figure 1. This ULM ultrasound image 29 provides a 3D anatomical mapping of the target body structure 10. This ULM ultrasound image 29 provides a very precise 3D mapping of the target body structure 10 of the patient. In order to obtain an ULM ultrasound image of a target body structure 10 like the brain vascular system 12, microbubbles are injected in the patient. Many 3D transcranial images are acquired. Microbubbles are localized and within a few minutes, a 3D ULM image is obtained.

This ULM ultrasound image 29 can be obtained prior to the monitoring of the target body structure 10 by the tracking system 19 or during the monitoring of the target body structure 10 by the system 19. In some embodiments, the memory 28 of the control unit 20 can store several ULM ultrasound images 29 of the target body structure 10. The memory 28 can thus store a succession of ULM ultrasound image 29 of the target body structure 10. In some embodiments, in order to reduce storage energy, each new ULM ultrasound image 29 replaces the prior one inside the memory 28. In order to increase the precision and accuracy of the mapping of the target body structure 10 during its monitoring by the system 19, the acquisition of an ULM ultrasound image 29 is done in real time. This provides a real time mapping of the target body structure 10 and enables to take quick structure changes into consideration. For example, the case where the device 15 is a catheter, navigating the catheter through the brain vascular system 12 deforms the blood vessels 14 to a certain degree and may shift some curvature or angle with regards to the ULM ultrasound image 29 acquired before inserting the device 15 into the target body structure 10. This real time mapping occurs in that a new acquisition of an ULM ultrasound image 29 is launched, by the control unit 20 as soon the prior acquisition of an ULM ultrasound image 29 is terminated, each new ULM ultrasound image 29 thus replacing the prior one as soon its acquisition is terminated.

The control unit 20 is also designed to display, on a screen 30, each acquired and/or stored ULM ultrasound image 29. This is illustrated on figure 2.

By combining the real time information obtained from each first probe 22 regarding the at least one tracker 26 and the information of the stored ULM ultrasound image 29, the control unit 20 is able to display, in real time, the localisation of the at least one tracker 26 on said ULM ultrasound image 29, as shown on figure 4. This enables the surgeon to know, precisely, where the device 15, and more particularly the steerable element 16 of the device 15 is, and how and to where it is to be manipulated (by means of the steering element 17) in order to reach the occlusion point (for example).

The system 19 thus enables to implement a device 15 tracking and localization method, wherein the method enables:
- the visualization, on the screen 30, of at least one ultrasound image 29, for example an ULM image 29, of the target body structure 10 of a patient,
- the real time tracking of the steerable element 16 of the device 15,
- the real time localization of said steerable element 16 inside the target body structure 10,
- the real time display, on the screen, 30 within the ultrasound image 29 of said steerable element 16 localization.

## Claims

1. A device tracking system (19) configured to monitor a target body structure (10) of a patient and localize a device inside said target body structure (10), the tracking system (19) comprising:
- a control unit (20) with a memory (28), the memory (28) being configured to store at least one ULM, that is Ultrasound Localisation Microscopy, ultrasound image (29) of the target body structure (10), the control unit (20) being configured to launch an acquisition of the least one ULM ultrasound image (29),
- a device (15) comprising:
∘ at least one steering element (17) configured to be handled outside the target body structure (10),
∘ at least one steerable element (16) configured to be introduced inside the target body structure (10), the steerable element (16) and the steering element (17) being configured to be physically connected by at least one connection element (18),
- at least one probe (22) configured to be brought in contact with a securing body part (24) of the patient, the securing body (24) part surrounding at least partially the target body structure (10), the at least one probe (22) being configured to be in real time communication with the control unit (20),
- at least one tracker (26) configured to be secured to the steerable element (16) of the device,
**wherein** the at least one probe (22) and the at least one tracker (26) are configured to communicate by means of ultrasounds and information collected as a result of this ultrasound communication is sent to the control unit (20), the control unit (20) being thus able to localize, in real time, the steerable element (16) inside the target body structure (10),
**wherein** the control unit (20) is further designed to display, on a screen (30), the at least one stored ULM ultrasound image (29) and display, in real time, the localisation of the steerable element (16) on said at least one ULM ultrasound image (29).

2. The system (19) according to claim 1, **wherein** the memory (28) of the control unit (20) is configured to store a succession of ULM ultrasound images (29) of the target body structure (10), each new ULM ultrasound image (29) replacing the prior one.

3. The system (19) according to the preceding claim, **wherein** the acquisition of an ULM ultrasound image (29) is done in real time, a new acquisition of an ULM ultrasound image (29) being launched as soon the prior acquisition of an ULM ultrasound image (29) is terminated, each new ULM ultrasound image (29) replacing the prior one as soon its acquisition is terminated.

4. The system (19) according to the preceding claim, **wherein** a new acquisition of an ULM ultrasound image (29) is launched, by the control unit (20) as soon the prior acquisition of an ULM ultrasound image (29) is terminated.

5. The system (19) according to any one of the preceding claims, **wherein** the control unit (20) is configured to combine the real-time information collected as a result of the ultrasound communication and the information of the stored ULM ultrasound image (29).

6. The system (19) according to any one of the preceding claims, **wherein** the control unit (20) is an emission/reception system configured to transform electric impulses into acoustic impulses and vice versa in order to enable acoustic characterization of a given environment, in particular the target body structure (10).

7. The system (19) according to any one of the preceding claims, wherein the at least one probe (22) is configured to be removably secured to the securing body part (24) of the patient, the securing body (24) part surrounding at least partially the target body structure (10).

8. The system (19) according to any one of the preceding claims, wherein the at least one probe (22) comprises at least one ultrasound transducer and the at least one tracker (26) comprises at least one ultrasound sensor.

9. The system (19) according to the preceding claim, wherein the at least one tracker (26) comprising an object strongly reflecting ultrasounds waves.

10. The system (19) according to claims **1** to **6,** wherein the at least one tracker (26) comprises at least one ultrasound transducer and the at least one probe (22) comprises at least one ultrasound sensor.

11. The system (19) according to the preceding claim wherein the at least one steerable element (16) measures less than 1cm in diameter, more particularly between 0,1 and 3mm in diameter.

12. The system (19) according to any one of the preceding claims, wherein the device is a catheter, the at least one steerable element (16) is a catheter tip, and the at least one steering element is a catheter handle.

13. The system (19) according to any one of the preceding claims, wherein the target body structure (10) is the vascular system (12) of the brain.

14. The system (19) according to any one of the preceding claims, wherein the system (16) is able to localize the steerable element (16) with a better precision than half the size of the wavelength of the ultrasound used to perform the localization.

## Patentansprüche

1. Vorrichtungs-Verfolgungssystem (19), das so konfiguriert ist, dass es eine Zielkörperstruktur (10) eines Patienten überwacht und eine Vorrichtung innerhalb der Zielkörperstruktur (10) lokalisiert, wobei das Verfolgungssystem (19) Folgendes umfasst:
- eine Steuereinheit (20) mit einem Speicher (28), wobei der Speicher (28) so konfiguriert ist, dass er mindestens ein ULM-Ultraschallbild (29) (wobei ULM für Ultraschall-Lokalisierungsmikroskopie steht) der Zielkörperstruktur (10) speichert, wobei die Steuereinheit (20) so konfiguriert ist, dass sie eine Erfassung des mindestens einen ULM-Ultraschallbilds (29) startet,
- eine Vorrichtung (15), die Folgendes umfasst:
∘ mindestens ein Steuerelement (17), das so konfiguriert ist, dass es außerhalb der Zielkörperstruktur (10) gehandhabt werden kann,
∘ mindestens ein steuerbares Element (16), das so konfiguriert ist, dass es in die Zielkörperstruktur (10) eingeführt werden kann, wobei das steuerbare Element (16) und das Steuerelement (17) so konfiguriert sind, dass sie durch mindestens ein Verbindungselement (18) physisch verbunden sind,
- mindestens eine Sonde (22), die so konfiguriert ist, dass sie mit einem befestigenden Körperteil (24) des Patienten in Kontakt gebracht werden kann, wobei der befestigende Körperteil (24) mindestens teilweise die Zielkörperstruktur (10) umgibt, wobei die mindestens eine Sonde (22) so konfiguriert ist, dass sie in Echtzeitkommunikation mit der Steuereinheit (20) steht,
- mindestens einen Tracker (26), der so konfiguriert ist, dass er an dem steuerbaren Element (16) der Vorrichtung befestigt werden kann,
**wobei** die mindestens eine Sonde (22) und der mindestens eine Tracker (26) so konfiguriert sind, dass sie mittels Ultraschall kommunizieren, und Informationen, die als Ergebnis dieser Ultraschallkommunikation gesammelt werden, an die Steuereinheit (20) gesendet werden, wobei die Steuereinheit (20) somit in der Lage ist, das steuerbare Element (16) innerhalb der Zielkörperstruktur (10) in Echtzeit zu lokalisieren,
**wobei** die Steuereinheit (20) ferner ausgebildet ist, auf einem Bildschirm (30) das mindestens eine gespeicherte ULM-Ultraschallbild (29) anzuzeigen und in Echtzeit die Lokalisierung des steuerbaren Elements (16) auf dem mindestens einen ULM-Ultraschallbild (29) anzuzeigen.

2. System (19) nach Anspruch 1, **wobei** der Speicher (28) der Steuereinheit (20) so konfiguriert ist, dass er eine Folge von Ultraschallbildern (29) der Zielkörperstruktur (10) speichert, wobei jedes neue Ultraschallbild (29) das vorherige ersetzt.

3. System (19) nach dem vorhergehenden Anspruch, **wobei** die Erfassung eines ULM-Ultraschallbilds (29) in Echtzeit erfolgt, wobei eine neue Erfassung eines ULM-Ultraschallbilds (29) gestartet wird, sobald die vorherige Erfassung eines ULM-Ultraschallbilds (29) beendet ist, wobei jedes neue ULM-Ultraschallbild (29) das vorherige ersetzt, sobald seine Erfassung beendet ist.

4. System (19) nach dem vorhergehenden Anspruch, **wobei** eine neue Erfassung eines ULM-Ultraschallbildes (29) durch die Steuereinheit (20) gestartet wird, sobald die vorherige Erfassung eines ULM-Ultraschallbildes (29) beendet ist.

5. System (19) nach einem der vorhergehenden Ansprüche, **wobei** die Steuereinheit (20) so konfiguriert ist, dass sie die als Ergebnis der Ultraschallkommunikation erhobenen Echtzeitinformationen und die Informationen des gespeicherten ULM-Ultraschallbilds (29) kombiniert.

6. System (19) nach einem der vorhergehenden Ansprüche, **wobei** die Steuereinheit (20) ein Sende-/Empfangssystem ist, das so konfiguriert ist, dass es elektrische Impulse in akustische Impulse umwandelt und umgekehrt, um eine akustische Charakterisierung einer bestimmten Umgebung, insbesondere der Zielkörperstruktur (10), zu ermöglichen.

7. System (19) nach einem der vorhergehenden Ansprüche, wobei die mindestens eine Sonde (22) so konfiguriert ist, dass sie abnehmbar an dem befestigenden Körperteil (24) des Patienten befestigt befestigt werden kann, wobei der befestigende Körperteil (24) mindestens teilweise die Zielkörperstruktur (10) umgibt.

8. System (19) nach einem der vorhergehenden Ansprüche, wobei die mindestens eine Sonde (22) mindestens einen Ultraschallwandler umfasst, und der mindestens eine Tracker (26) mindestens einen Ultraschallsensor umfasst.

9. System (19) nach dem vorhergehenden Anspruch, wobei der mindestens eine Tracker (26) ein Objekt umfasst, das Ultraschallwellen stark reflektiert.

10. System (19) nach den Ansprüchen **1** bis **6,** wobei der mindestens eine Tracker (26) mindestens einen Ultraschallwandler umfasst, und die mindestens eine Sonde (22) mindestens einen Ultraschallsensor umfasst.

11. System (19) nach dem vorhergehenden Anspruch, wobei das mindestens eine steuerbare Element (16) weniger als 1 cm Durchmesser, insbesondere zwischen 0,1 und 3 mm Durchmesser, misst.

12. System (19) nach einem der vorhergehenden Ansprüche, wobei die Vorrichtung ein Katheter ist, das mindestens eine steuerbare Element (16) eine Katheterspitze ist und das mindestens eine Steuerelement ein Kathetergriff ist.

13. System (19) nach einem der vorhergehenden Ansprüche, wobei die Zielkörperstruktur (10) das vaskuläre System (12) des Gehirns ist.

14. System (19) nach einem der vorhergehenden Ansprüche, wobei das System (16) in der Lage ist, das steuerbare Element (16) mit einer Präzision zu lokalisieren, die besser ist als die Hälfte der Wellenlänge des Ultraschalls, der verwendet wird, um die Lokalisierung durchzuführen.

## Revendications

1. Système de suivi (19) d'un dispositif configuré pour surveiller une structure corporelle cible (10) d'un patient et localiser un dispositif à l'intérieur de ladite structure corporelle cible (10), le système de suivi (19) comprenant :
- une unité de commande (20) avec une mémoire (28), la mémoire (28) étant configurée pour stocker au moins une image ultrasonore ULM (Ultrasound Localisation Microscopy) (29) de la structure corporelle cible (10), l'unité de commande (20) étant configurée pour lancer une acquisition de l'au moins une image ultrasonore ULM (29),
- un dispositif (15) comprenant :
- au moins un élément de guidage (17) configuré pour être manipulé à l'extérieur de la structure corporelle cible (10),
- au moins un élément orientable (16) configuré pour être introduit à l'intérieur de la structure corporelle cible (10), l'élément orientable (16) et l'élément de guidage (17) étant configurés pour être physiquement reliés par au moins un élément de connexion (18),
- au moins une sonde (22) configurée pour être mise en contact avec une zone de fixation (24) du corps du patient, la zone de fixation (24) entourant au moins partiellement la structure corporelle cible (10), l'au moins une sonde (22) étant configurée pour être en communication, en temps reel, avec l'unité de commande (20),
- au moins un traceur (26) configuré pour être fixé à l'élément orientable (16) du dispositif,
l'au moins une sonde (22) et l'au moins un traceur (26) étant configurés pour communiquer au moyen d'ultrasons et les informations collectées à la suite de cette communication par ultrasons sont envoyées à l'unité de commande (20), l'unité de commande (20) étant ainsi capable de localiser, en temps réel, l'élément orientable (16) à l'intérieur de la structure corporelle cible (10),
l'unité de commande (20) étant en outre conçue pour afficher, sur un écran (30), la au moins une image ultrasonore ULM stockée (29) et afficher, en temps réel, la localisation de l'élément orientable (16) sur ladite au moins une image ultrasonore ULM (29).

2. Le système (19) selon la revendication 1, dans lequel la mémoire (28) de l'unité de commande (20) est configurée pour stocker une succession d'images ultrasonores ULM (29) de la structure corporelle cible (10), chaque nouvelle image ultrasonore ULM (29) remplaçant la précédente.

3. Système (19) selon la revendication précédente, dans lequel l'acquisition d'une image ultrasonore ULM (29) est effectuée en temps réel, une nouvelle acquisition d'une image ultrasonore ULM (29) étant lancée dès que l'acquisition précédente d'une image ultrasonore ULM (29) est terminée, chaque nouvelle image ultrasonore ULM (29) remplaçant la précédente dès que son acquisition est terminée.

4. Le système (19) selon la revendication précédente, dans lequel une nouvelle acquisition d'une image ultrasonore ULM (29) est lancée par l'unité de commande (20) dès que l'acquisition précédente d'une image ultrasonore ULM (29) est terminée.

5. Le système (19) selon l'une quelconque des revendications précédentes, dans lequel l'unité de commande (20) est configurée pour combiner les informations en temps réel collectées en résultat de la communication par ultrasons et les informations de l'image ultrasonore ULM stockée (29).

6. Le système (19) selon l'une quelconque des revendications précédentes, dans lequel l'unité de commande (20) est un système d'émission/réception configuré pour transformer des impulsions électriques en impulsions acoustiques et vice versa afin de permettre la caractérisation acoustique d'un environnement donné, en particulier la structure corporelle cible (10).

7. Le système (19) selon l'une quelconque des revendications précédentes, dans lequel l'au moins une sonde (22) est configurée pour être fixée de manière amovible à la zone de fixation (24) du corps du patient, la zone de fixation (24) entourant au moins partiellement la structure corporelle cible (10).

8. Le système (19) selon l'une quelconque des revendications précédentes, dans lequel l'au moins une sonde (22) comprend au moins un transducteur à ultrasons et l'au moins un traceur (26) comprend au moins un capteur à ultrasons.

9. Le système (19) selon la revendication précédente, dans lequel l'au moins un traceur (26) comprend un objet réfléchissant fortement les ondes ultrasonores.

10. Le système (19) selon les revendications 1 à 6, dans lequel l'au moins un traceur (26) comprennd au moins un transducteur à ultrasons et l'au moins une sonde (22) comprend au moins un capteur à ultrasons.

11. Le système (19) selon la revendication précédente, dans lequel l'au moins un élément orientable (16) mesure moins de 1 cm de diamètre, plus particulièrement entre 0,1 et 3 mm de diamètre.

12. Le système (19) selon l'une quelconque des revendications précédentes, dans lequel le dispositif est un cathéter, l'au moins un élément orientable (16) est une pointe de cathéter, et l'au moins un élément d'orientation est une poignée de cathéter.

13. Le système (19) selon l'une quelconque des revendications précédentes, dans lequel la structure corporelle cible (10) est le système vasculaire (12) du cerveau.

14. Le système (19) selon l'une quelconque des revendications précédentes, dans lequel le système (19) est capable de localiser l'élément orientable (16) avec une précision supérieure à la moitié de la longueur d'onde des ultrasons utilisés pour effectuer la localisation.
